# EUROPEAN PATENT APPLICATION

(11) **EP 3 440 989 A1**
(43) Date of publication of application: **13.02.2019**
(21) Application number: 17185478.9
(22) Date of filing: 09.08.2017
(51) Int. Cl.: A61B 3/12, A61B 3/117, A61B 3/15

(54) **SYSTEM FOR IMAGING AN EYE**

(71) Applicant: Koninklijke Philips N.V., 5656 AE Eindhoven (NL)
(72) Inventor: SCHMEITZ, Harold Agnes Wilhelmus, 5656 AE Eindhoven (NL); DE BRUIJN, Frederik Jan, 5656 AE Eindhoven (NL); JUTTE, Petrus Theodorus, 5656 AE Eindhoven (NL); HADDEMAN, Theodoor Bastiaan Johannes, 5656 AE Eindhoven (NL)
(74) Representative: de Haan, Poul Erik

(57) **Abstract**

There is provided a system (200) for imaging an eye (202). The system comprises a light source (204) for illuminating the eye and optics (206) for adapting one or both of an optical path length of light of a first wavelength range reflected from the posterior segment (F) of the eye to focus that light and an optical path length of light of a second wavelength range reflected from the anterior segment (E) of the eye to focus that light. The second wavelength range is different from the first wavelength range. The system also comprises at least one imaging sensor (208) configured to acquire one or more of an image of the posterior segment (F') from light of the first wavelength range reflected from the posterior segment and an image of the anterior segment (E') from light of the second wavelength range reflected from the anterior segment.

## Description

### TECHNICAL FIELD OF THE INVENTION

The invention relates to the field of optics and, in particular, to a system for imaging an eye of a subject and a method of operating the system.

### BACKGROUND TO THE INVENTION

Various aspects of the human eye are known to reflect physical or physiological changes to, or conditions of, other parts of the body. For example, the fundus of the eye includes a light sensitive (or photosensitive), image-capturing tissue referred to as the "retina", which is known to exhibit a variety of characteristics that make it a valuable source of diagnostic information for eye-related diseases (such as glaucoma), as well as other diseases of the body (such as diabetes). The retina of the eye is known to respond to physical or physiological changes of the entire body. Some of these changes are visible or measurable, and are thus helpful in the early detection of symptoms and diagnosis diseases. The fundus of the eye also includes other parts that can provide useful information on the health of a subject, such as the optic disc, blood vessels, nerve fiber layer, and so on.

A unique feature of the ocular fundus is that it offers a relatively obstruction-free view of various arteries and veins, without being inhibited by occluding and/or scattering layers such as the skin. This enables ophthalmologists to check for the presence of vascular anomalies, such as aneurisms and even neovascularization. Additionally, the unobstructed view on the ocular blood vessels poses an advantage in assessment of the levels of various metabolic compounds carried by the vascular system by exploiting the differences in characteristic absorption of visible and invisible ultraviolet or infrared light.

Traditionally, an ophthalmologist examines the eye using an ophthalmoscope, which provides a direct view of a fragment of the fundus under coaxial illumination from a built-in light source. Ophthalmoscopes can take various forms and can be used for direct observation of the fundus or for indirect observation of the fundus by using a relay lens that is held separately near the eye. Ophthalmoscopes for direct observation of the fundus (such as classical ophthalmoscopes or the newer "panoptic" ophthalmoscopes) are typically held relatively close to the eye and, in some instances, a portion of the ophthalmoscope may even rest softly on the orbital region of the eye for support. In contrast, ophthalmoscopes for indirect observation of the fundus can be used at a remote distance from the eye but these ophthalmoscopes still require a handheld relay lens to be held close to the eye.

Therefore, existing techniques for both direct and indirect fundus imaging (for example, using an ophthalmoscope) are obtrusive. The existing techniques are also typically only performed by trained professionals in a medical setting, which means that eyes are seldom examined for fundus symptoms other than during the occasional eye test for glasses or the Amsler test for macular degeneration or other vision problems. Consequently, markers of various diseases that may be observable in the eyes often remain undetected.

With the advances in chemical and digital photography, the ophthalmoscope has evolved into the fundus camera. The fundus camera is useful for fundus imaging in cases where only the backside of the fundus needs to be captured and not the peripheral fundus area. Some fundus cameras use a line-wise scanning method based on laser illumination. Other fundus cameras that are used to compose a fundus image include planar silicon image sensors, which are based on charge-coupled devices (CCDs), or complementary metal-oxide semiconductor (CMOS) imaging sensors.

However, as with the more traditional ophthalmoscopes mentioned earlier, the position of existing fundus cameras required for fundus imaging means that the fundus camera can be obtrusive and also the lens of existing fundus cameras needs to be precisely aligned manually to the pupil to minimize the field of view at the fundus, which limits the use of fundus cameras to trained and experienced operators.

There is thus a need for an improved system for imaging an eye of a subject.

### SUMMARY OF THE INVENTION

As noted above, a limitation with existing techniques for imaging an eye of a subject is that they require precise manual alignment of the imaging sensor with the pupil and they can also be obtrusive.

It would thus be beneficial to relax the alignment constraints that are imposed in existing eye imaging systems to ease the use of the systems such that they may be operated easily, even by untrained users, and to also allow imaging at larger distances such that imaging performed using the systems is not obtrusive. Moreover, imaging at a larger distance will mean that any orientation of the eye (and thus the pupil position) will fall within an image acquired by the imaging sensor.

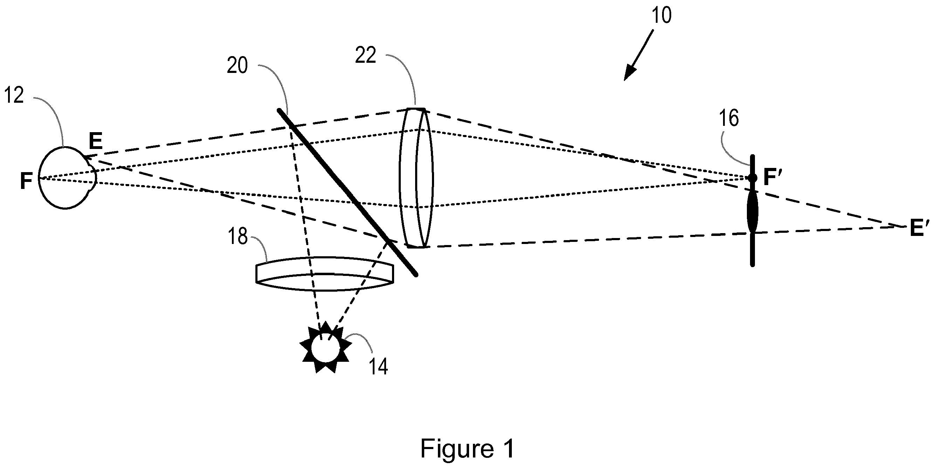

However, although it would be advantageous to image at larger distances for these reasons, there are also further problems that need to be overcome. For example, imaging at a larger distance will result in a smaller field of view of the fundus. Moreover, even though the pupil will also be in view at the imaging plane, it will not be possible to acquire a sharp image of both the posterior segment (for example, the fundus) of the eye and the anterior segment (for example, the pupil) of the eye. This problem is apparent from Figure 1, for example, where it can be seen that a sharp image of the posterior segment of the eye can only be acquired in the plane of point F', whereas a sharp image of the anterior segment of the eye can only be acquired in the plane of point E'. Thus, if the anterior segment of the eye E is imaged in the plane of point F', the anterior segment will be imaged out of focus and a large blur radius will result.

It would thus be valuable to have an improved system for imaging an eye of a subject, which overcomes the problems described above.

Therefore, according to a first aspect of the invention, there is provided a system for imaging an eye of a subject. The system comprises a light source for illuminating the eye with light and optics for adapting one or both of an optical path length of light of a first wavelength range reflected from the posterior segment of the eye to focus the light of the first wavelength range and an optical path length of light of a second wavelength range reflected from the anterior segment of the eye to focus the light of the second wavelength range. The second wavelength range is different from the first wavelength range. The system also comprises at least one imaging sensor configured to acquire one or more of an image of the posterior segment of the eye from light of the first wavelength range reflected from the posterior segment of the eye and an image of the anterior segment of the eye from light of the second wavelength range reflected from the anterior segment of the eye.

In some embodiments, the at least one imaging sensor may be a single imaging sensor that is configured to acquire the image of the posterior segment of the eye from light of the first wavelength range reflected from the posterior segment of the eye and the image of the anterior segment of the eye from light of the second wavelength range reflected from the anterior segment of the eye.

In some embodiments, light of the first wavelength range reflected from the posterior segment of the eye and light of the second wavelength range reflected from the anterior segment of the eye may focus on the same plane at the at least one imaging sensor.

In some embodiments, the image of the posterior segment of the eye and the image of the anterior segment of the eye may be acquired at a remote distance from the eye. In some embodiments, the image of the posterior segment of the eye and the image of the anterior segment of the eye may be acquired at the same distance from the eye. In some embodiments, the image of the posterior segment of the eye and the image of the anterior segment of the eye may be acquired simultaneously.

In some embodiments, the image of the posterior segment of the eye may be acquired from light of the first wavelength range reflected from the posterior segment of the eye by filtering light to allow through light of the first wavelength and/or the image of the anterior segment of the eye may be acquired from light of the second wavelength range reflected from the anterior segment of the eye by filtering light to allow through light of the second wavelength. In some embodiments, the first wavelength range may be the red wavelength range and/or the green wavelength range, and/or the second wavelength range may be the blue wavelength range and/or the infra-red wavelength range.

In some embodiments, the optics may comprise a beam splitter for splitting the light into the light of the first wavelength range reflected from the posterior segment of the eye and the light of the second wavelength range reflected from the anterior segment of the eye, before the light passes to the at least one imaging sensor.

In some embodiments, the optics may comprise a diffraction grating for generating, before the light passes to the at least one imaging sensor, any one or more of a focus shift for the light of the second wavelength range reflected from the anterior segment of the eye and a tilt of the light of the second wavelength range reflected from the anterior segment of the eye.

In some embodiments, the optics may comprise a first aperture through which light of the first wavelength range reflected from the posterior segment of the eye passes to the at least one imaging sensor and a second aperture through which light of the second wavelength range reflected from the anterior segment of the eye passes to the at least one imaging sensor. In some embodiments, the second aperture may be smaller than the first aperture.

In some embodiments, the at least one imaging sensor that acquires the image of the anterior segment of the eye may be configured to acquire a sequence of images of the anterior segment of the eye to track movement of the eye. In some embodiments, the at least one imaging sensor may be configured to combine the sequence of images of the anterior segment of the eye to form a single image of the anterior segment of the eye with a larger field of view than the individual images in the sequence.

In some embodiments, the posterior segment of the eye may comprise the fundus or retina of the eye and/or the anterior segment of the eye may comprise the iris and/or the pupil of the eye.

According to a second aspect of the invention, there is provided a method of operating a system to image an eye of a subject. The method comprises illuminating the eye with light from a light source and adapting, by optics, one or both of an optical path length of light of a first wavelength range reflected from the posterior segment of the eye to focus the light of the first wavelength range and an optical path length of light of a second wavelength range reflected from the anterior segment of the eye to focus the light of the second wavelength range. The second wavelength range is different from the first wavelength range. The method also comprises acquiring, by at least one imaging sensor, one or more of an image of the posterior segment of the eye from light of the first wavelength range reflected from the posterior segment of the eye and an image of the anterior segment of the eye from light of the second wavelength range reflected from the anterior segment of the eye.

According to a third aspect of the invention, there is provided a computer program product comprising a computer readable medium, the computer readable medium having computer readable code embodied therein, the computer readable code being configured such that, on execution by a suitable computer or processor, the computer or processor is caused to perform the method described above.

According to the aspects and embodiments described above, the limitations of existing techniques are addressed. In particular, according to the above-described aspects and embodiments, an unobtrusive (and, for example, automated) imaging system is provided by which imaging of an eye of a subject can be performed at a remote distance from the eye, such that the system may operate without physical contact to the subject.

At the same time, the system does not compromise on the quality of images that can be produced. Instead, the image quality is improved by the system focusing light of one wavelength to acquire high quality images of the posterior segment of the eye and focusing light of another wavelength to acquire high quality images the anterior of the eye. Thus, the system improves the focus outside the captured area of the posterior segment of the eye, such that it is possible to acquire a sharp image of the anterior segment of the eye as well as a sharp image of the posterior segment of the eye. This can be useful, for example, where pupil tracking is required as pupil tracking will be more precise using sharper images of the anterior segment of the eye. Moreover, it is possible to acquire sharper images of the eye without imposing alignment constraints on the system to achieve the sharp images, which means that high quality images of the eye can be acquired easily, even by an untrained user.

There is thus provided an improved system for imaging an eye of a subject, which overcomes the existing problems.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is an illustration of a system for imaging an eye of a subject;
Figure 2 is an illustration of an example system for imaging an eye of a subject according to an embodiment;
Figure 3 is an illustration of an example of optics according to an embodiment;
Figure 4 is an illustration of an example system for imaging an eye of a subject according to another embodiment;
Figure 5 is an illustration of an example system for imaging an eye of a subject according to another embodiment;
Figure 6 is an illustration of an example system for imaging an eye of a subject according to another embodiment;
Figure 7 is an illustration of an example system for imaging an eye of a subject according to another embodiment;
Figure 8 is a flow chart illustrating a method of operating a system to image an eye of a subject according to an embodiment.

### DETAILED DESCRIPTION OF THE EMBODIMENTS

As mentioned earlier, in existing systems, it is not possible to acquire (or capture) sharp images of the posterior and anterior segments of the eye unobtrusively, at a remote distance from the eye, without constraints being imposed on the precise alignment of the imaging sensor with the pupil of the eye.

Figure 1 illustrates a system 10 for imaging an eye 12 of a subject in which the problem of acquiring sharp images of the eye at a remote distance is apparent. With reference to Figure 1, the system 10 comprises a light source 14 for illuminating the eye 12 of the subject with light. The system 10 also comprises an imaging sensor 16 configured to acquire an image of the posterior segment F of the eye from light that is reflected from the posterior segment F of the eye 12 and an image of the anterior segment E of the eye from light of that is reflected from the anterior segment E of the eye 12. As illustrated in Figure 1, a lens 18 that is positioned in front of the light source 14 converges light emitted by the light source 14 to illuminate the eye 12, a mirror 20 that is angled at the opposite side of the lens 18 to the light source 14 reflects the light emitted by the light source 14 toward the eye 12 and this light is then reflected from the eye 12, namely from both the posterior segment F and the anterior segment E of the eye. The light reflected from the eye 12 of the subject passes through the mirror 20 and is focused by a further lens 22 toward the imaging sensor 16.

However, as is apparent from Figure 1, although the posterior segment F of the eye 12 of the subject is in focus in the image of the posterior segment F' of the eye 12 captured by the imaging sensor 16, the anterior segment E of the eye 12 of the subject is out of focus in this image and would only be in focus in an image of anterior segment E' of the eye 12 of the subject captured at a further distance from the eye of the subject. The applicants have recognized that the posterior segment F of the eye 12 and the anterior segment E of the eye 12 have different focus planes, which are illustrated at points F' and E' respectively in Figure 1. With this insight, the applicants have arrived at an improved system that can acquire sharper images of both the posterior segment F of the eye 12 and the anterior segment E of the eye 12.

In particular, the system for imaging an eye of a subject described herein improves the focus outside of the captured posterior segment F. As noted earlier, the improved system overcomes the existing problems. In particular, the improved system allows for the unobtrusive (and, for example, automated) acquisition of a sharp image of the eye at a remote distance from the eye, where one or more of a posterior segment of the eye and an anterior segment of the eye are in focus in the image, without the need for precise alignment of the system to achieve the sharp image.

Briefly, the improved system for imaging an eye of a subject comprises a light source for illuminating the eye with light and optics for adapting one or both of an optical path length of light of a first wavelength range reflected from the posterior segment of the eye to focus the light of the first wavelength range and an optical path length of light of a second wavelength range reflected from the anterior segment of the eye to focus the light of the second wavelength range.

The second wavelength range is different from the first wavelength range. For example, in any of the embodiments described herein, the first wavelength range may be the red wavelength range (for example, a wavelength range between 620 nm and 750 nm), the green wavelength range (for example, a wavelength range between 495 nm and 570), or the red and green wavelength range (for example, a wavelength range between 495 nm and 750 nm. On the other hand, the second wavelength range may be the violet and blue wavelength range (for example, a wavelength range between 380 nm and 495 nm) or the near infra-red or infra-red wavelength range (for example, a wavelength range between 750 nm and 950 nm).

The posterior segment of the eye referred to herein can, for example, comprise the fundus of the eye, or any other posterior segment of the eye, or any combination of posterior segments of the eye. The fundus of the eye comprises the retina (which itself comprises the macula and fovea), the optic disc, blood vessels, and nerve fiber layer, as well as various hidden tissues such as the choroidal layer. Thus, the posterior segment of the eye referred to herein can comprise the retina, the optic disc, the macula, the fovea, one or more blood vessels, the nerve fiber layer, and/or any other features of the fundus. The anterior segment of the eye referred to herein can, for example, comprise any one or more of the iris of the eye, the pupil of the eye, and any other anterior segment of the eye, or any combination of anterior segments of the eye.

In addition to the light source and the optics described earlier, the system for imaging an eye of a subject also comprises at least one imaging sensor configured to acquire one or more of an image of the posterior segment of the eye from light of the first wavelength range reflected from the posterior segment of the and an image of the anterior segment of the eye from light of the second wavelength range reflected from the anterior segment of the eye. According to any of the embodiments herein, the at least one imaging sensor may, for example, comprise a camera, a charge-coupled device (CCD) imaging sensor, a complementary metal-oxide semiconductor (CMOS) imaging sensor, or any other imaging sensor, or any combination of imaging sensors, suitable for imaging the eye. In some embodiments, the imaging sensor may be an RGB color imaging sensor. In other embodiments, the imaging sensor can be a monochrome imaging sensor.

In embodiments that use light of the imperceptible near infra-red or infra-red wavelength range for imaging the eye, an imaging sensor with a sensitivity beyond the visible light spectrum can be used and it is possible to perform unnoticeable measurements. Also, in embodiments that use light of the blue wavelength range, it is possible to use existing imaging sensors, since the blue channel is often unused or at least contains minimal information in these sensors. For example, although some typical imaging sensors utilize RGB color filters to capture a full-color image, it is often only the green, or the red and green, channels that are used for diagnosis.

In some embodiments, light of the first wavelength range reflected from the posterior segment of the eye and light of the second wavelength range reflected from the anterior segment of the eye may focus on the same plane (for example, the same imaging plane) at the at least one imaging sensor. For example, in some embodiments, the light of the first wavelength range reflected from the posterior segment of the eye and light of the second wavelength range reflected from the anterior segment of the eye may focus on different imaging sensors, which are in the same plane, or on the same imaging sensor in the same plane.

In other embodiments, light of the first wavelength range reflected from the posterior segment of the eye and light of the second wavelength range reflected from the anterior segment of the eye may focus on a different plane (for example, a different imaging plane) at the at least one imaging sensor. For example, in some embodiments, the light of the first wavelength range reflected from the posterior segment of the eye and light of the second wavelength range reflected from the anterior segment of the eye may focus on different imaging sensors, which are in a different planes, or on the same imaging sensor in a different plane.

In any of the embodiments described herein, in addition to the light source, optics and at last one imaging sensor mentioned earlier, the system may also comprise other components. For example, the system may comprise one or more lenses, one or more mirrors, one or more other optical components, a control unit, one or more memories, one or more audio components (such as one or more speakers), one or more visual components (such as one or more display screens), or any other component, or any combination of components. Any one or more of the components of the system may be operably coupled together via one or more wired or wireless pathways (which can, for example, be data/electrical/communication pathways, such as one or more buses).

In some embodiments, one or more components of the system may be controllable by a control unit of the system that is operably coupled to the one or more components. In embodiments where the system comprises a control unit, the control unit can comprise one or more processors, processing units, multi-core processors or modules that are configured or programmed to control one or more components of the system. For example, in some embodiments, the control unit may be configured to execute instructions stored in memory to control one or more components of the system. In other embodiments, the control unit may comprise logic such as a field-programmable gate array ("FPGA"), an application-specific integrated circuit ("ASIC"), or any other types of logic, or any combination of types of logic, and the logic may be configured or programmed to control one or more components of the system. Thus, the control unit can comprise a plurality of software and/or hardware modules that are configured to control one or more components of the system. In various embodiments, the control unit may be communicatively coupled with one or more remote computing devices, such as via one or more networks. The one or more networks may include one or more local area networks, wide area networks (such as the Internet), one or more personal area networks (such as Bluetooth), or similar.

In any of the embodiments described herein, the image of the posterior segment of the eye and the image of the anterior segment of the eye can be acquired at a remote distance from the eye using the described systems. A remote distance from the eye can be, for example, a distance that is larger than the distance possible to acquire suitably sharp images of the eye using existing equipment. For example, in some embodiments, the distance between the eye and the at least one imaging sensor may be a distance equal to 10 centimeters, or a distance greater than 10 centimeters (for example, 15 centimeters, 30 centimeters, or any other distance). However, it will be understood that other distances between the eye and the at least one imaging sensor are also possible. In some embodiments, the image of the posterior segment of the eye and the image of the anterior segment of the eye may be acquired at the same distance from the eye. In other embodiments, the image of the posterior segment of the eye and the image of the anterior segment of the eye may be acquired at a different distance from the eye.

The system according to any of the embodiments described herein is unobtrusive. In some embodiments, the system may also be at least partially (or fully) automated.

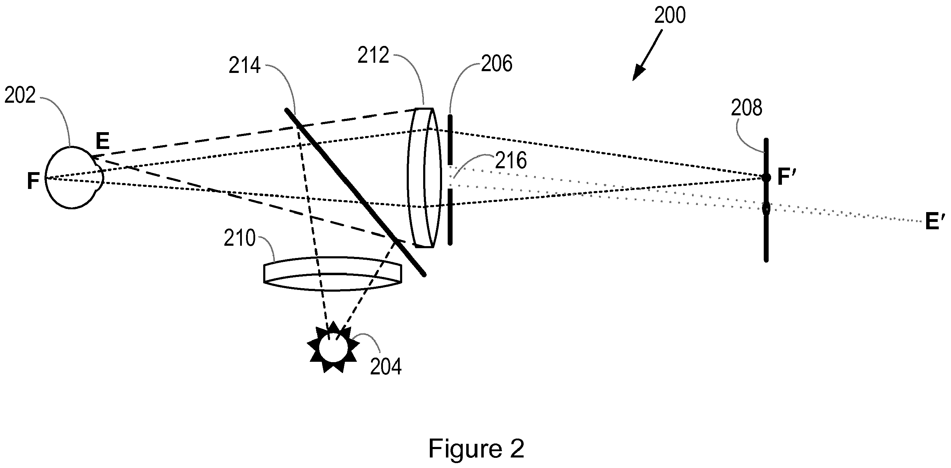

Figure 2 illustrates an example of the system 200 for imaging an eye 202 of a subject (not depicted) according to an embodiment. With reference to Figure 2, the system 200 comprises a light source 204 for illuminating the eye 202 with light. More specifically, the light source 204 is configured to emit light to illuminate the eye 202 of the subject.

The system 200 also comprises optics 206 for adapting an optical path length of light. In particular, the optics 206 are for adapting an optical path length of light of a second wavelength range reflected from the anterior segment E of the eye 202 to focus the light of the second wavelength range. Alternatively or in addition, the optics 206 can be for adapting an optical path length of light of a first wavelength range reflected from the posterior segment F of the eye 202 to focus the light of the first wavelength range. The second wavelength range is different from the first wavelength range.

The system 200 also comprises at least one imaging sensor 208 configured to acquire one or more of an image of the posterior segment F' of the eye 202 from light of the first wavelength range reflected from the posterior segment F of the eye 202 and an image of the anterior segment E' of the eye 202 from light of the second wavelength range reflected from the anterior segment E of the eye 202. In the example embodiment illustrated in Figure 2, the system 200 comprises a single imaging sensor 208. However, it will be understood that, in other embodiments, the system 200 may comprise any number of multiple imaging sensors.

In the illustrated example embodiment of Figure 2, the optics 206 for adapting an optical path length of light comprise a color-coded aperture (for example, a color filter) 216. The color-coded aperture 216 is configured to split the light reflected from the eye 202 of the subject into different color channels, namely into light of the first wavelength range and light of the second wavelength range. For example, different parts of the color-coded aperture 216 can allow light of different wavelength ranges to pass through the color-coded aperture 216. The color-coded aperture 216 may also be referred to as a color-dependent aperture.

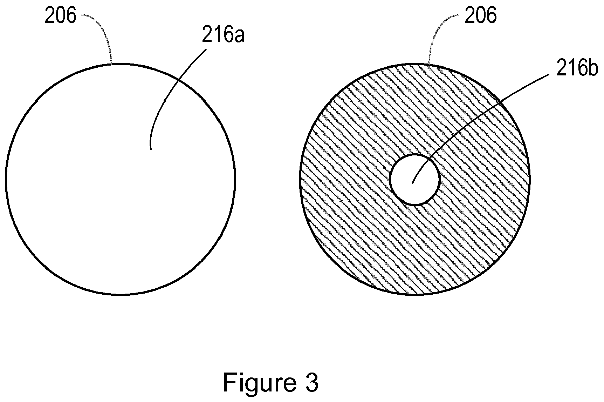

Figure 3 illustrates an example of such optics 206, namely a color-coded aperture 216, according to an embodiment. As illustrated in Figure 3, the optics 206 comprise a first aperture 216a through which light of the first wavelength range reflected from the posterior segment F of the eye 202 passes to the imaging sensor 208 and a second aperture 216b through which light of the second wavelength range reflected from the anterior segment E of the eye 202 passes to the imaging sensor 208.

The second aperture 216b is smaller than the first aperture 216a. In this way, the light of the second wavelength range reflected from the anterior segment E of the eye 202 passes through the smaller aperture 216b to allow a sharp image of the anterior segment E' of the eye 202 to be acquired by the imaging sensor 208. On the other hand, the light of the first wavelength range reflected from the posterior segment F of the eye 202 passes through the larger aperture 216a (and, optionally, also the smaller aperture 216b) to allow a sharp image of the posterior segment F' of the eye 202 to be acquired by the imaging sensor 208. In some embodiments, the first aperture 216a and the second aperture 216b may be in the form of a filter. For example, the filter can comprise a transparent circular center, which forms the smaller aperture 216b, and a color-coded outer radius (for example, surrounding the transparent circular center), which together with the transparent circular center forms the larger aperture 216a. The color-coded outer radius may allow only light of the first wavelength range to pass through and may block light of the second wavelength range. Thus, light of the second wavelength range may be confined to passing through the transparent circular center of the filter, whereas light of the first wavelength can pass through the entire filter.

The light of the first wavelength range reflected from the posterior segment F of the eye 202 passes through the larger first aperture 216a to maximize the light collection and field of view. Thus, the light collection and field of view can be maximized for the color channel of the first wavelength range. On the other hand, the light of the second wavelength range reflected from the anterior segment E of the eye 202 passes only through the smaller second aperture 216b to obtain a larger depth of field (DOF). Thus, a larger DOF can be created for the color channel of the second wavelength range. Although the anterior segment E of the eye 202 is not perfectly focused at the point F' at which an image of the anterior segment E' of the eye 202 can be acquired, the blur radius of the image of the anterior segment E' of the eye 202 is significantly reduced.

In an alternative embodiment, instead of a color-coded aperture, the optics 206 of Figure 2 may comprise a mechanism for forming (or a mechanism configured to form) different apertures in a time-sequential manner. For example, the mechanism may be a digital micro-mirror device (DMD), a liquid crystal display (LCD), or any other mechanism suitable for forming different apertures in a time-sequential manner. In these embodiments, the light source 204 may be controllable to illuminate the eye 202 with a color sequence of light and the time sequence of apertures may be synchronized with the color sequence of light. Thus, the light source 204 of the system 200 according to these embodiments may be a color-sequential light source. The imaging sensor 208 of the system 200 according to these embodiments can comprise a monochrome sensor, since the wavelength of light can be encoded by the light source 204.

Returning back to Figure 2, according to some embodiments, the system 200 may also comprise other components. For example, in some embodiments, the system 200 may comprise one or more optical elements 210 through which light emitted by the light source 204 passes to illuminate the eye 202 of the subject. The one or more optical elements 210 can, for example, converge light emitted by the light source 204 to illuminate the eye 202 of the subject. The one or more optical elements 210 may, for example, comprise one or more achromatic lens combinations, or other refractive elements such as Fresnel lenses, one or more reflective optical elements such as converging mirrors, one or more wavelength selective filters, one or more optical diffusing elements, one or more optical polarizing elements, or any other optical elements, or combination of optical elements, suitable to pass light emitted by the light source 204 to illuminate the eye 202 of the subject.

Alternatively or in addition, in some embodiments, the system 200 can comprise one or more further optical elements 212 through which light reflected from the eye 202 passes before reaching the imaging sensor 208. The one or more further optical elements 212 can, for example, focus light reflected from the eye 202 on the imaging sensor 208. The one or more further optical elements 212 may, for example, comprise one or more achromatic lens combinations, one or more reflective optical elements such as converging mirrors, one or more wavelength selective filters, one or more diffractive elements, one or more optical polarizing elements, or any other optics components, or combination of optics components suitable to focus light reflected from the eye 202 on the imaging sensor 208.

Alternatively or in addition, in some embodiments, the system 200 can comprise one or more mirrors 214 angled to reflect light emitted by the light source 204. The one or more mirrors 214 can, for example, reflect light emitted by the light source 204 toward the eye 202 of the subject. The one or more further mirrors 214 may, for example, comprise one or more semi-transparent mirrors, or any other type of mirrors, or any combination of mirrors, suitable to reflect light emitted by the light source 204.

For example, in the illustrated example of Figure 2, an optical element 210 (such as a lens) for converging light is positioned in front of the light source 204. The optical element 210 converges light emitted by the light source 204 to illuminate the eye 202 of the subject. A mirror 214 is angled at the opposite side of the optical element 210 to the light source 204 to reflect the light emitted by the light source 204 toward the eye 202 of the subject. Thus, the light emitted by the light source 204 is converged by the optical element 210 and the converged light is reflected by the mirror 214 toward the eye 202 of the subject. The light reflected by the mirror 214 toward the eye 202 of the subject is then reflected from the eye 202. More specifically, as illustrated in Figure 2, the light is reflected from the posterior segment F of the eye 202 and the anterior segment E of the eye 202. The mirror 214 is a semi-transparent mirror in the illustrated example embodiment of Figure 2. Thus, the light that is reflected from the eye 202 of the subject passes through the mirror 214. A further optical element 212 (such as a further lens) is positioned at the opposite side of the mirror 214 to the eye 202 of the subject. The further optical element 212 focuses the light that passes through the mirror 214 toward the imaging sensor 208 via the optics 206, which adapt the optical path length of light in the manner described earlier.

In the illustrated example embodiment of Figure 2, the optics 206 for adapting the optical path length of light are positioned between the eye 202 of the subject and the imaging sensor 208. More specifically, in the example system 200 illustrated in Figure 2, the optics 206 are positioned between the optical element 212 that focuses light and the imaging sensor 208. In this way, the light that is focused by the optical element 212 passes through the optics 206 before reaching the imaging sensor 208. According to the example system 200 of Figure 2, it is possible to ensure that both the posterior segment F of the eye 202 and the anterior segment E of the eye 202 are in focus (or at least as sharp as possible) in an image acquired by the imaging sensor 208.

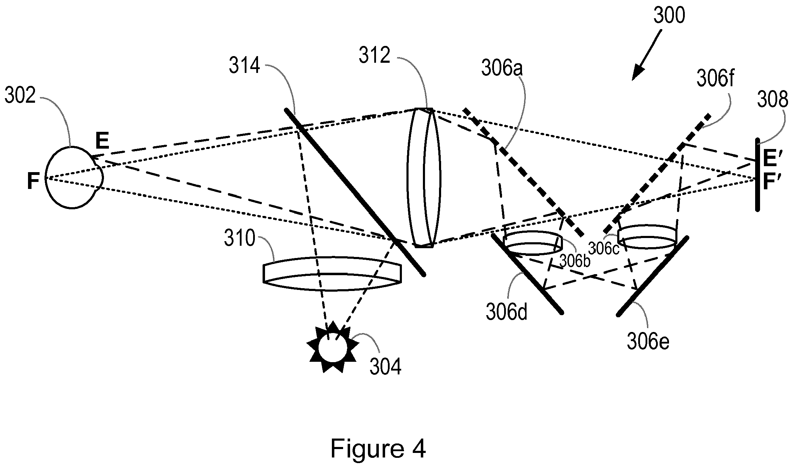

Figure 4 illustrates an example of the system 300 for imaging an eye 302 of a subject (not depicted) according to another embodiment. With reference to Figure 4, the system 300 comprises a light source 304 for illuminating the eye 302 with light. More specifically, the light source 304 is configured to emit light to illuminate the eye 302 of the subject.

The system 300 also comprises optics 306a, 306b, 306c, 306d, 306e, 306f for adapting an optical path length of light. In particular, the optics 306a, 306b, 306c, 306d, 306e, 306f are for adapting an optical path length of light of a second wavelength range reflected from the anterior segment E of the eye 302 to focus the light of the second wavelength range. Alternatively or in addition, the optics 306a, 306b, 306c, 306d, 306e, 306f can be for adapting an optical path length of light of a first wavelength range reflected from the posterior segment F of the eye 302 to focus the light of the first wavelength range. The second wavelength range is different from the first wavelength range.

The system 300 also comprises at least one imaging sensor 308 configured to acquire one or more of an image of the posterior segment F' of the eye 302 from light of the first wavelength range reflected from the posterior segment F of the eye 302 and an image of the anterior segment E' of the eye 302 from light of the second wavelength range reflected from the anterior segment E of the eye 302. In the example embodiment illustrated in Figure 4, the system 300 comprises a single imaging sensor 308. However, it will be understood that, in other embodiments, the system 300 may comprise any number of multiple sensors.

In the illustrated example embodiment of Figure 4, the optics 306a, 306b, 306c, 306d, 306e, 306f for adapting an optical path length of light comprise a beam splitter 306a for splitting the light into the light of the first wavelength range reflected from the posterior segment F of the eye 302 and the light of the second wavelength range reflected from the anterior segment E of the eye 302, before the light passes to the imaging sensor 308. In some embodiments, for example, the beam splitter 306a may comprise a dichroic beam splitter, or any combination of beam splitters, suitable to split the light in the manner described. The light of the first wavelength range reflected from the posterior segment F of the eye 302 passes through the beam splitter 306a before reaching the imaging sensor 308. On the other hand, the light of the second wavelength range reflected from the anterior segment E of the eye 302 is reflected by the beam splitter 306a before reaching the imaging sensor 308.

In some embodiments, as illustrated in Figure 4, the optics 306a, 306b, 306c, 306d, 306e, 306f of the system 300 can comprise other components such as one or more relay lenses 306b, 306c to transfer light to one or more other components in the system 300, one or more mirrors 306d, 306e to reflect light toward one or more other components in the system 300, and/or one or more further beam splitters 306f to split light in the system 300. The one or more relay lenses 306b, 306c may comprise, for example, achromatic lens combinations and other refractive elements, such as prisms, one or more refractive elements, one or more polarization filters, or any other type of optical element, or any combination of optical elements, suitable to relay light to one or more other components in the system 300. The one or more mirrors 306d, 306e may comprise, for example, one or more mirrors, flat mirrors or curved mirrors, fully reflective mirrors, or partially reflective (semi-transparent) mirrors or beam splitters, dichroic mirrors or beam splitters, prisms, or any other optical elements, or combination of optical elements, suitable to reflect light toward one or more other components in the system 300. The one or more further beam splitters 306f may comprise, for example, one or more dichroic semi-transparent mirrors, or any other type of beam splitter, or any combination of beam splitters, suitable to split light in the system 300.

In the illustrated example embodiment of Figure 4, any one or more of the beam splitters 306a, 306f and any one or more of the mirrors can be oriented under 45 degrees with respect to the optical axis of the system 300. The one or more beam splitters 306a, 306f and the one or more mirrors have the function of folding the optical path of the light reflected from the anterior E of the eye to a point E' in the plane of the imaging sensor 308, such that the anterior segment E of the eye 302 appears in focus on the imaging sensor 308.

In operation, the light of the first wavelength range reflected from the posterior segment F of the eye 302 that passes through the beam splitter 306a also passes through the further beam splitter 306f before reaching the imaging sensor 308. Also, the light of the second wavelength range reflected from the anterior segment E of the eye 302 that is reflected by the beam splitter 306a is reflected toward a first relay lens 306b, passes through the first relay lens 306b toward a first mirror 306d, is reflected by the first mirror 306d toward a second mirror 306e, is reflected by the second mirror 306e toward a second relay lens 306c, passes through the second relay lens 306c to the further beam splitter 306f and is reflected by the further beam splitter 306f toward the imaging sensor 308.

According to some embodiments, the system 300 may also comprise other components. For example, in some embodiments, the system 300 can comprise one or more optical elements 310 through which light emitted by the light source 304 passes to illuminate the eye 302 of the subject (such as any of those optical elements 210 described earlier with reference to Figure 2), one or more further optical elements 312 through which light reflected from the eye 302 passes before reaching the imaging sensor 308 (such as any of those further optical elements 212 described earlier with reference to Figure 2), and/or one or more mirrors 314 angled to reflect light emitted by the light source 304 (such as any of those mirrors 214 described earlier with reference to Figure 2). For example, in the illustrated example of Figure 4, an optical element 310, a further optical element 312, and a mirror 314 are arranged and operate in the manner described earlier in respect of the optical element 210, further optical element 212, and mirror 214 of Figure 2 respectively, which will not be repeated here but will be understood to apply.

In the illustrated example embodiment of Figure 4, the optics 306a, 306b, 306c, 306d, 306e, 306f for adapting the optical path length of light are positioned between the eye 302 of the subject and the imaging sensor 308. More specifically, in the example system 300 illustrated in Figure 4, the optics 306a, 306b, 306c, 306d, 306e, 306f are positioned between the optical element 312 that focuses light and the imaging sensor 308. In this way, the light that is focused by the optical element 312 passes through the optics 306a, 306b, 306c, 306d, 306e, 306f before reaching the imaging sensor 308. As mentioned earlier, the optics 306a, 306b, 306c, 306d, 306e, 306f comprise a beam splitter 306a. As illustrated in Figure 4, the beam splitter 306a is positioned at the opposite side of the optical element 312 to the eye 302 and is angled relative to the optical element 312. According to the system 300 of Figure 4, it is possible to ensure that both the posterior segment F of the eye 302 and the anterior segment E of the eye 302 are in focus in an image acquired by the imaging sensor 308.

In embodiments where the system 300 comprises one more further optical elements 312, the one or more relay lenses 306b and 306c can be used shorten or lengthen the optical path length of the light reflected from the anterior E of the eye between the one or more further optical elements 312 and the imaging sensor 308, such that the image of the anterior segment E' of the eye 102 coincides with the plane of the imaging sensor 308 and the anterior segment E of the eye 302 appears in focus on the imaging sensor 308.

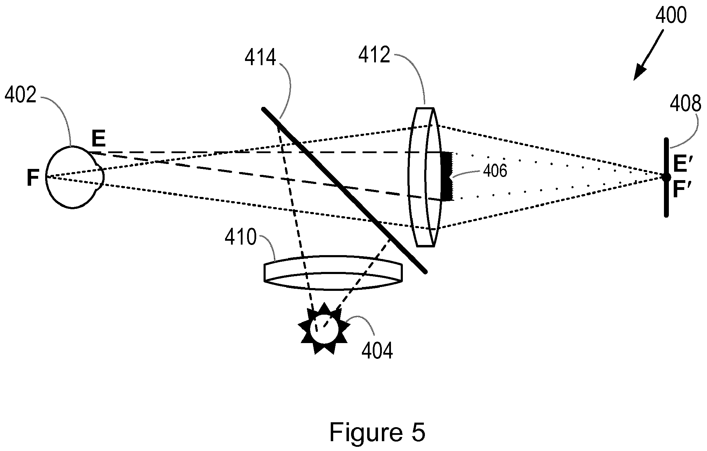

Figure 5 illustrates an example of the system 400 for imaging an eye 402 of a subject (not depicted) according to another embodiment. With reference to Figure 5, the system 400 comprises a light source 404 for illuminating the eye 402 with light. More specifically, the light source 404 is configured to emit light to illuminate the eye 402 of the subject.

The system 400 also comprises optics 406 for adapting an optical path length of light. In particular, the optics 406 are for adapting an optical path length of light of a second wavelength range reflected from the anterior segment E of the eye 402 to focus the light of the second wavelength range. Alternatively or in addition, the optics 406 can be for adapting an optical path length of light of a first wavelength range reflected from the posterior segment F of the eye 402 to focus the light of the first wavelength range. The second wavelength range is different from the first wavelength range.

The system 400 also comprises at least one imaging sensor 408 configured to acquire one or more of an image of the posterior segment F' of the eye 402 from light of the first wavelength range reflected from the posterior segment F of the eye 402 and an image of the anterior segment E' of the eye 402 from light of the second wavelength range reflected from the anterior segment E of the eye 402. In the example embodiment illustrated in Figure 5, the system 400 comprises a single imaging sensor 408. However, it will be understood that in other embodiments, the system 500 may comprise any number of multiple sensors.

In the illustrated example embodiment of Figure 5, the optics 406 for adapting an optical path length of light comprise a diffraction grating 406 for generating, before the light passes to the imaging sensor 408, a focus shift for the light of the second wavelength range reflected from the anterior segment E of the eye 402. The diffraction grating 406 can, for example, function like a Fresnel lens. Thus, in the system 500 of Figure 5, the optical path length of light of the second wavelength range reflected from the anterior segment E of the eye 402 is adapted by the diffraction grating 406. The diffraction grating 406 is positioned in the center of an optical element (such as a lens) 412 and thus the optical path length of light of the second wavelength range reflected from the anterior segment E of the eye 402 is adapted in the center. As the diffraction grating 406 is positioned on the center of the optical element 412, it is possible to image the surface of the anterior segment E of the eye E on the center of the imaging sensor 408.

According to some embodiments, the system 400 may also comprise other components. For example, in some embodiments, the system 400 can comprise one or more optical elements 410 through which light emitted by the light source 404 passes to illuminate the eye 402 of the subject (such as any of those optical elements 210 described earlier with reference to Figure 2), one or more further optical elements 412 through which light reflected from the eye 402 passes before reaching the imaging sensor 408 (such as any of those further optical elements 212 described earlier with reference to Figure 2), and/or one or more mirrors 414 angled to reflect light emitted by the light source 404 (such as any of those mirrors 214 described earlier with reference to Figure 2). For example, in the illustrated example of Figure 5, an optical element 410, a further optical element 412, and a mirror 414 are arranged and operate in the manner described earlier in respect of the optical element 210, further optical element 212, and mirror 214 of Figure 2 respectively, which will not be repeated here but will be understood to apply.

In the illustrated example embodiment of Figure 5, the optics 406 for adapting the optical path length of light are positioned between the eye 402 of the subject and the imaging sensor 408. More specifically, in the example system 400 illustrated in Figure 5, the optics 406 are positioned between the optical element 412 that focuses light and the imaging sensor 408. In this way, the light that is focused by the optical element 412 passes through the optics 406 before reaching the imaging sensor 408. According to the system 400 of Figure 5, it is possible to ensure that both the posterior segment F of the eye 502 and the anterior segment E of the eye 502 are in focus in an image acquired by the imaging sensor 508.

Although not illustrated, in another embodiment, the optics 406 of the system 400 of Figure 5 may further comprise a color-coded aperture 206 (as described earlier with reference to Figure 2, which will not be repeated here but will be understood to apply) in combination with a diffraction grating 406 (as described with reference to Figure 5, which will not be repeated here but will be understood to apply). In embodiments comprising a combination of the color-coded aperture 206 and the diffraction grating 406, the color-coded aperture may be positioned between the optical element 412 and the diffraction grating 406.

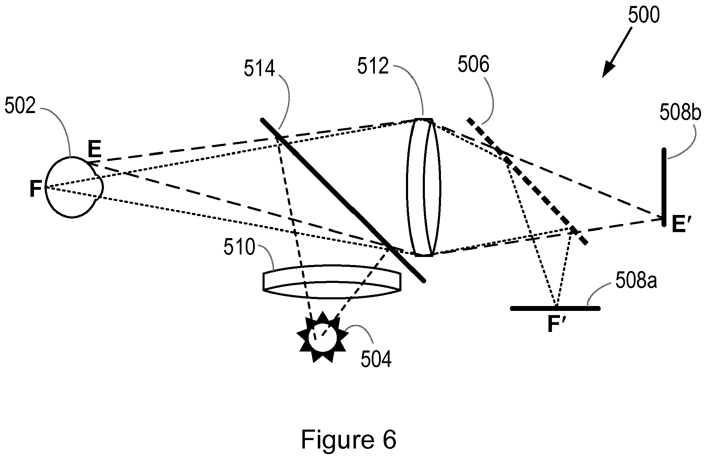

Figure 6 illustrates an example of the system 500 for imaging an eye 502 of a subject (not depicted) according to another embodiment. With reference to Figure 5, the system 500 comprises a light source 504 for illuminating the eye 502 with light. More specifically, the light source 504 is configured to emit light to illuminate the eye 502 of the subject.

The system 500 also comprises optics 506 for adapting an optical path length of light. In particular, the optics 506 are for adapting an optical path length of light of a first wavelength range reflected from the posterior segment F of the eye 502 to focus the light of the first wavelength range. Alternatively or in addition, the optics 506 can be for adapting an optical path length of light of a second wavelength range reflected from the anterior segment E of the eye 502 to focus the light of the second wavelength range. The second wavelength range is different from the first wavelength range.

The system 500 also comprises at least one imaging sensor 508a, 508b configured to acquire one or more of an image of the posterior segment F' of the eye 502 from light of the first wavelength range reflected from the posterior segment F of the eye 502 and an image of the anterior segment E' of the eye 502 from light of the second wavelength range reflected from the anterior segment E of the eye 502. In the example embodiment illustrated in Figure 6, the system 500 comprises two imaging sensors 508a, 508b. However, it will be understood that in other embodiments, the system 500 may comprise a single imaging sensor or any other number of multiple sensors. Also, in the example embodiment illustrated in Figure 6, the imaging sensors 508a, 508b are each positioned in a different plane (for example, a different imaging plane). However, it will be understood that in other embodiments comprising multiple imaging sensors, at least two imaging sensors or all imaging sensors may be positioned in the same plane (for example, the same imaging plane).

In the illustrated example embodiment of Figure 6, the optics 506 for adapting an optical path length of light comprise a beam splitter 506 for splitting the light into the light of the first wavelength range reflected from the posterior segment F of the eye 502 and the light of the second wavelength range reflected from the anterior segment E of the eye 502, before the light passes to the at least one imaging sensor 508a, 508b. For example, the light of the second wavelength range reflected from the anterior segment E of the eye 502 passes through the beam splitter 506 before reaching a first (or primary) imaging sensor 508a. On the other hand, the light of the first wavelength range reflected from the posterior segment F of the eye 502 is reflected by the beam splitter 506 before reaching a second (or secondary) imaging sensor 508b. In some embodiments, the beam splitter 506 may comprise a dichroic beam splitter, or any other type of beam splitter, or any combination of beam splitters, suitable to split the light in the manner described.

According to some embodiments, the system 500 may also comprise other components. For example, in some embodiments, the system 500 can comprise one or more optical elements 510 through which light emitted by the light source 504 passes to illuminate the eye 502 of the subject (such as any of those optical elements 210 described earlier with reference to Figure 2), one or more further optical elements 512 through which light reflected from the eye 502 passes before reaching the at least one imaging sensor 508 (such as any of those further optical elements 212 described earlier with reference to Figure 2), and/or one or more mirrors 514 angled to reflect light emitted by the light source 504 (such as any of those mirrors 214 described earlier with reference to Figure 2). For example, in the illustrated example of Figure 6, an optical elements 510, a further optical element 512, and a mirror 514 are arranged and operate in the manner described earlier in respect of the optical element 210, further optical element 212, and mirror 214 of Figure 2 respectively, which will not be repeated here but will be understood to apply.

The optics 506 for adapting the optical path length of light are positioned between the eye 502 of the subject and the imaging sensors 508a, 508b. More specifically, in the example system 500 illustrated in Figure 6, the optics 506 are positioned between the optical element 512 that focuses light and the imaging sensors 508a, 508b. In this way, the light that is focused by the optical element 512 passes through the optics 506 before reaching the imaging sensors 508a, 508b. As mentioned earlier, the optics 506 in the illustrated example embodiment of Figure 6 comprise a beam splitter 506. As illustrated in Figure 6, the beam splitter 506 is positioned at the opposite side of the optical element 512 to the eye 502 and is angled relative to the optical element 512. According to the system 500 of Figure 6, it is possible to ensure that the posterior segment F of the eye 502 is in focus in an image acquired by the first imaging sensor 508a and the anterior segment E of the eye 502 is in focus in an image acquired by the second imaging sensor 508b.

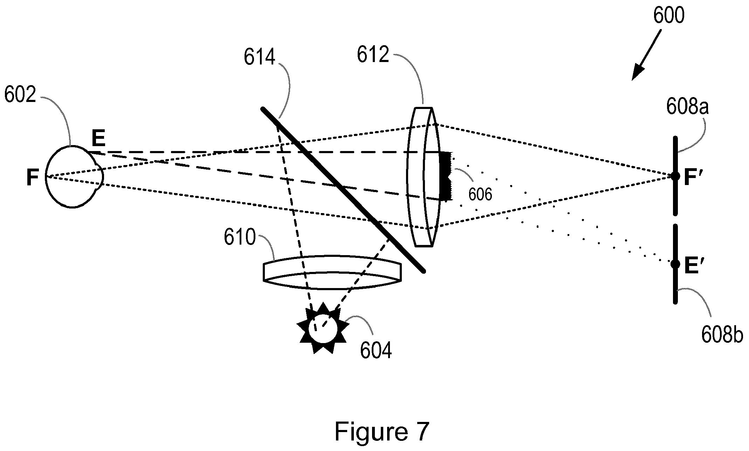

Figure 7 illustrates an example of the system 600 for imaging an eye 602 of a subject (not depicted) according to another embodiment. With reference to Figure 6, the system 600 comprises a light source 604 for illuminating the eye 602 with light. More specifically, the light source 604 is configured to emit light to illuminate the eye 602 of the subject.

The system 600 also comprises optics 606 for adapting an optical path length of light. In particular, the optics 606 are for adapting an optical path length of light of a first wavelength range reflected from the posterior segment F of the eye 602 to focus the light of the first wavelength range. Alternatively or in addition, the optics 606 can be for adapting an optical path length of light of a second wavelength range reflected from the anterior segment E of the eye 602 to focus the light of the second wavelength range. The second wavelength range is different from the first wavelength range.

The system 600 also comprises at least one imaging sensor 608a, 608b configured to acquire one or more of an image of the posterior segment F' of the eye 602 from light of the first wavelength range reflected from the posterior segment F of the eye 602 and an image of the anterior segment E' of the eye 602 from light of the second wavelength range reflected from the anterior segment E of the eye 602. In the example embodiment illustrated in Figure 7, the system 600 comprises two imaging sensors 608a, 608b. However, it will be understood that in other embodiments, the system 600 may comprise a single imaging sensor or any other number of multiple sensors. Also, in the example embodiment illustrated in Figure 7, the imaging sensors 608a, 608b are each positioned in the same plane (for example, in the same imaging plane). However, it will be understood that in other embodiments comprising multiple imaging sensors, at least two imaging sensors or all imaging sensors may be positioned in a different plane (for example, in a different imaging plane).

In the illustrated example embodiment of Figure 7, the optics 606 for adapting an optical path length of light comprise a diffraction grating 606 for generating, before the light passes to the imaging sensors 608a, 608b, a focus shift for the light of the second wavelength range reflected from the anterior segment E of the eye 602 and a tilt of the light of the second wavelength range reflected from the anterior segment E of the eye 602. Thus, in the system 600 of Figure 7, the optical path length of light of the second wavelength range reflected from the anterior segment E of the eye 602 is adapted by the diffraction grating 606. The diffraction grating 606 is positioned in the center of the optical element 612 and thus the optical path length of light of the second wavelength range reflected from the anterior segment E of the eye 602 is adapted in the center.

In the illustrated example embodiment of Figure 7, the light of the first wavelength range reflected from the posterior segment F of the eye 602 is received at a first (or primary) imaging sensor 608a. On the other hand, the light of the second wavelength range reflected from the anterior segment E of the eye 602, which is adapted by the diffraction grating 606 in the manner described earlier, is received at a second (or secondary) imaging sensor 608b. In effect, the diffraction grating 606 deflects the light of the second wavelength range reflected from the anterior segment E of the eye 602 onto the second imaging sensor 608b. In this illustrated example embodiment, the first imaging sensor 608a is different from the second imaging sensor 608b. However, the first imaging sensor 608a and the second imaging sensor 608b are positioned in the same plane (for example, the same imaging plane) in this illustrated example embodiment, which is possible since the diffraction grating 606 not only generates a tilt of the light of the second wavelength range reflected from the anterior segment E of the eye 602 but also generates a focus shift of the light of the second wavelength range reflected from the anterior segment E of the eye 602.

According to some embodiments, the system 600 may also comprise other components. For example, in some embodiments, the system 600 can comprise one or more optical elements 610 through which light emitted by the light source 604 passes to illuminate the eye 602 of the subject (such as any of those optical elements 210 described earlier with reference to Figure 2), one or more further optical elements 612 through which light reflected from the eye 602 passes before reaching the imaging sensors 608a, 608b (such as any of those further optical elements 212 described earlier with reference to Figure 2), and/or one or more mirrors 614 angled to reflect light emitted by the light source 604 (such as any of those mirrors 214 described earlier with reference to Figure 2). For example, in the illustrated example of Figure 7, an optical element 610, a further optical element 612, and a mirror 614 are arranged and operate in the manner described earlier in respect of the optical element 210, further optical element 212, and mirror 214 of Figure 2 respectively, which will not be repeated here but will be understood to apply.

In the illustrated example embodiment of Figure 7, the optics 606 for adapting the optical path length of light are positioned between the eye 602 of the subject and the imaging sensors 608a, 608b. More specifically, in the example system 600 illustrated in Figure 7, the optics 606 are positioned between the optical element 612 that focuses light and the imaging sensors 608a, 608b. In this way, the light that is focused by the optical element 612 passes through the optics 606 before reaching the imaging sensors 608a, 608b. According to the system 600 of Figure 7, it is possible to ensure that the posterior segment F of the eye 602 is in focus in an image acquired by the first imaging sensor 608a and the anterior segment E of the eye 602 is in focus in an image acquired by the second imaging sensor 608b.

In any of the embodiments described herein, the image of the posterior segment F' of the eye 202, 302, 402, 502, 602 and the image of the anterior segment E' of the eye 202, 302, 402, 502, 602 can be acquired simultaneously. Alternatively, the image of the posterior segment F' of the eye 202, 302, 402, 502, 602 and the image of the anterior segment E' of the eye 202, 302, 402, 502, 602 can be acquired at different times. In some embodiments, the image of the posterior segment F' of the eye 202, 302, 402, 502, 602 may be acquired from light of the first wavelength range reflected from the posterior segment F of the eye 202, 302, 402, 502, 602 by filtering light to allow through light of the first wavelength. Alternatively or in addition, the image of the anterior segment E' of the eye 202, 302, 402, 502, 602 may be acquired from light of the second wavelength range reflected from the anterior segment E of the eye 202, 302, 402, 502, 602 by filtering light to allow through light of the second wavelength.

In any of the embodiments described herein, the at least one imaging sensor 208, 308, 408, 508a, 508b, 608a, 608b that acquires the image of the anterior segment E' of the eye 202, 302, 402, 502, 602 of the subject can be configured to acquire a sequence of images (or video stream) of the anterior segment E' of the eye 202, 302, 402, 502, 602 to track movement of the eye 202, 302, 402, 502, 602. In these embodiments, the at least one imaging sensor 208, 308, 408, 508a, 508b, 608a, 608b may also be configured to combine (for example, stitch together) the sequence of images of the anterior segment E' of the eye 202, 302, 402, 502, 602 to form a single image of the anterior segment E' of the eye 202, 302, 402, 502, 602 with a larger field of view than the individual images in the sequence. Thus, multiple small field of view images can be combined to form a single image with larger field of view. For example, the field of view can be increased by stitching of the multiple images of the anterior segment E' of the eye 202, 302, 402, 502, 602 of the eye in the sequence in order to compose an image covering a wider area. This overcomes the drawback of a smaller field of view of the posterior segment when imaging at a larger imaging distance, since different parts of the posterior segment can be imaged in the sequence of images as the eye moves around to obtain a larger field of view of the posterior segment.

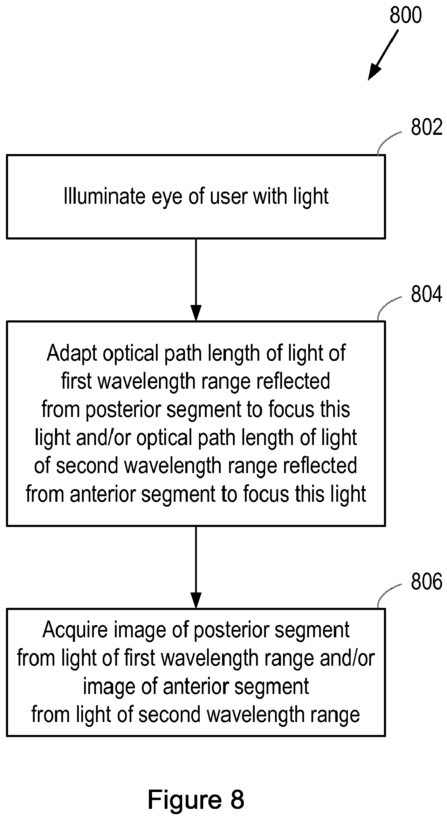

Figure 8 illustrates a method 800 of operating a system 200, 300, 400, 500, 600 to image an eye 202, 302, 402, 502, 602 of a subject according to an embodiment. The method 800 is for use in operating the systems 200, 300, 400, 500, 600 according to any of the embodiments described herein.

As illustrated in Figure 8, at block 802, the method comprises illuminating the eye 202, 302, 402, 502, 602 of the subject with light from a light source 204, 304, 404, 504, 604. At block 804 of Figure 8, an optical path length of light of a first wavelength range reflected from the posterior segment F of the eye 202, 302, 402, 502, 602 is adapted by optics 206, 306a, 306b, 306c, 306d, 306e, 306f, 406, 506, 606 to focus the light of the first wavelength range and/or an optical path length of light of a second wavelength range reflected from the anterior segment E of the eye 202, 302, 402, 502, 602 is adapted by optics 206, 306a, 306b, 306c, 306d, 306e, 306f, 406, 506, 606 to focus the light of the second wavelength range. The second wavelength range is different from the first wavelength range.

At block 806 of Figure 8, one or more of an image of the posterior segment F' of the eye 202, 302, 402, 502, 602 is acquired by at least one imaging sensor 208, 308, 408, 508a, 508b, 608a, 608b from light of the first wavelength range reflected from the posterior segment F of the eye 202, 302, 402, 502, 602 and an image of the anterior segment E' of the eye 202, 302, 402, 502, 602 is acquired by at least one imaging sensor 208, 308, 408, 508a, 508b, 608a, 608b from light of the second wavelength range reflected from the anterior segment E of the eye 202, 302, 402, 502, 602.

There is therefore provided herein an improved system for imaging an eye of a subject and also a method of operating the system to image an eye of a subject. The system described herein can be used for unobtrusive (and, for example, automated) health monitoring on the basis of images captured of the eye or, more specifically, the fundus of the eye. As such, the system can be used in the home (for example, in the bathroom) as part of a home-health monitoring system. The system may be used for early detection or monitoring of a physical or physiological condition or to follow deterioration. This can, for example be useful in respect of subjects suffering from retinopathy, such as that caused by diabetes or hypertension. In such applications, the acquired images can be made available to a medical professional to enable the early detection or monitoring in a remote manner, such as via the internet. In another application, the system may be used for remote spectral monitoring of metabolite concentrations dissolved in the blood, in order to report the concentrations to a user (for example, the subject themselves, an ophthalmologist, a medical professional, or any other user) or to store the concentrations as measurements as part of a health monitoring system.

There is further provided a computer program product comprising a computer readable medium, the computer readable medium having computer readable code embodied therein, the computer readable code being configured such that, on execution by a suitable computer or processor, the computer or processor is caused to perform the methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (200, 300, 400, 500, 600) for imaging an eye (202, 302, 402, 502, 602) of a subject, the system comprising:
a light source (204, 304, 404, 504, 604) for illuminating the eye (202, 302, 402, 502, 602) with light;
optics (206, 306a, 306b, 306c, 306d, 306e, 306f, 406, 506, 606) for adapting one or both of:
an optical path length of light of a first wavelength range reflected from the posterior segment (F) of the eye (202, 302, 402, 502, 602) to focus the light of the first wavelength range; and
an optical path length of light of a second wavelength range reflected from the anterior segment (E) of the eye (202, 302, 402, 502, 602) to focus the light of the second wavelength range, wherein the second wavelength range is different from the first wavelength range; and
at least one imaging sensor (208, 308, 408, 508a, 508b, 608a, 608b) configured to acquire one or more of an image of the posterior segment (F') of the eye (202, 302, 402, 502, 602) from light of the first wavelength range reflected from the posterior segment (F) of the eye (202, 302, 402, 502, 602) and an image of the anterior segment (E') of the eye (202, 302, 402, 502, 602) from light of the second wavelength range reflected from the anterior segment (E) of the eye (202, 302, 402, 502, 602).

2. A system (200, 300, 400) as claimed in claim 1, wherein the at least one imaging sensor (208, 308, 408) is a single imaging sensor that is configured to:
acquire the image of the posterior segment (F') of the eye (202, 302, 402) from light of the first wavelength range reflected from the posterior segment (F) of the eye (202, 302, 402) and the image of the anterior segment (E') of the eye (202, 302, 402) from light of the second wavelength range reflected from the anterior segment (E) of the eye (202, 302, 402).

3. A system (300, 400, 600) as claimed in any one of claims 1 or 2, wherein light of the first wavelength range reflected from the posterior segment (F) of the eye (302, 402, 602) and light of the second wavelength range reflected from the anterior segment (E) of the eye (302, 402, 602) focus on the same plane at the at least one imaging sensor (308, 408, 608a, 608b).

4. A system (200, 300, 400, 500, 600) as claimed in any one of the preceding claims, wherein the image of the posterior segment (F') of the eye (202, 302, 402, 502, 602) and the image of the anterior segment (E') of the eye (202, 302, 402, 502, 602) are acquired at a remote distance from the eye (202, 302, 402, 502, 602).

5. A system (300, 400, 600) as claimed in any one of the preceding claims, wherein the image of the posterior segment (F') of the eye (302, 402, 602) and the image of the anterior segment (E') of the eye (302, 402, 602) are acquired at the same distance from the eye (302, 402, 602).

6. A system (200, 300, 400, 500, 600) as claimed in any one of the preceding claims, wherein the image of the posterior segment (F') of the eye (202, 302, 402, 502, 602) and the image of the anterior segment (E') of the eye (202, 302, 402, 502, 602) are acquired simultaneously.

7. A system (200, 300, 400, 500, 600) as claimed in any one of the preceding claims, wherein:
the image of the posterior segment (F') of the eye (202, 302, 402, 502, 602) is acquired from light of the first wavelength range reflected from the posterior segment (F) of the eye (202, 302, 402, 502, 602) by filtering light to allow through light of the first wavelength; and/or
the image of the anterior segment (E') of the eye (202, 302, 402, 502, 602) is acquired from light of the second wavelength range reflected from the anterior segment (E) of the eye (202, 302, 402, 502, 602) by filtering light to allow through light of the second wavelength.

8. A system (200, 300, 400, 500, 600) as claimed in any one of the preceding claims, wherein:
the first wavelength range is the red wavelength range and/or the green wavelength range; and/or
the second wavelength range is the blue wavelength range or the infra-red wavelength range.

9. A system (300, 506) as claimed in any one of the preceding claims, wherein the optics comprise:
a beam splitter (306a, 506) for splitting the light into the light of the first wavelength range reflected from the posterior segment (F) of the eye (302, 502) and the light of the second wavelength range reflected from the anterior segment (E) of the eye (302, 502), before the light passes to the at least one imaging sensor (308, 508a, 508b).

10. A system (400, 600) as claimed in any one of the preceding claims, wherein the optics comprise:
a diffraction grating (406, 606) for generating, before the light passes to the at least one imaging sensor (408, 608a, 608b), any one or more of a focus shift for the light of the second wavelength range reflected from the anterior segment (E) of the eye (402, 602) and a tilt of the light of the second wavelength range reflected from the anterior segment (E) of the eye (402, 602).

11. A system (200) as claimed in any one of claims 1 to 8, wherein the optics (206) comprise:
a first aperture (208a) through which light of the first wavelength range reflected from the posterior segment (F) of the eye (202) passes to the at least one imaging sensor (208); and
a second aperture (208b) through which light of the second wavelength range reflected from the anterior segment (E) of the eye (202) passes to the at least one imaging sensor (208),
wherein the second aperture (208b) is smaller than the first aperture (208a).

12. A system (200, 300, 400, 500, 600) as claimed in any one of the preceding claims, wherein the at least one imaging sensor (208, 308, 408, 508a, 508b, 608a, 608b) that acquires the image of the anterior segment (E') of the eye (202, 302, 402, 502, 602) is configured to acquire a sequence of images of the anterior segment (E') of the eye (202, 302, 402, 502, 602) to track movement of the eye (202, 302, 402, 502, 602).

13. A system (200, 300, 400, 500, 600) as claimed in claim 12, wherein the at least one imaging sensor (208, 308, 408, 508a, 508b, 608a, 608b) is configured to combine the sequence of images of the anterior segment (E') of the eye (202, 302, 402, 502, 602) to form a single image of the anterior segment (E') of the eye (202, 302, 402, 502, 602) with a larger field of view than the individual images in the sequence.

14. A system (200, 300, 400, 500, 600) as claimed in any one of the preceding claims, wherein:
the posterior segment (F) of the eye (202, 302, 402, 502, 602) comprises the fundus or retina of the eye (202, 302, 402, 502, 602); and/or
the anterior segment (E) of the eye (202, 302, 402, 502, 602) comprises the iris and/or the pupil of the eye (202, 302, 402, 502, 602).

15. A method of operating a system (200, 300, 400, 500, 600) to image an eye (202, 302, 402, 502, 602) of a subject, the method comprising:
illuminating the eye (202, 302, 402, 502, 602) with light from a light source (204, 304, 404, 504, 604);
adapting, by optics (206, 306a, 306b, 306c, 306d, 306e, 306f, 406, 506, 606) one or both of:
an optical path length of light of a first wavelength range reflected from the posterior segment (F) of the eye (202, 302, 402, 502, 602) to focus the light of the first wavelength range; and
an optical path length of light of a second wavelength range reflected from the anterior segment (E) of the eye (202, 302, 402, 502, 602) to focus the light of the second wavelength range, wherein the second wavelength range is different from the first wavelength range; and
acquiring, by at least one imaging sensor (208, 308, 408, 508a, 508b, 608a, 608b), one or more of an image of the posterior segment (F') of the eye (202, 302, 402, 502, 602) from light of the first wavelength range reflected from the posterior segment (F) of the eye (202, 302, 402, 502, 602) and an image of the anterior segment (E') of the eye (202, 302, 402, 502, 602) from light of the second wavelength range reflected from the anterior segment (E) of the eye (202, 302, 402, 502, 602).
